# EUROPEAN PATENT SPECIFICATION

(11) **EP 1 073 440 B1**
(45) Date of publication and mention of the grant of the patent: **20.10.2004**
(21) Application number: 99918666.1
(22) Date of filing: 20.04.1999
(51) Int. Cl.: C07D 209/22, A61K 31/535, A61P 29/00

(54) **sPLA 2 INHIBITOR ESTERS**
INHIBITORISCHE ESTER FÜR sPLA 2
ESTERS INHIBITEURS DE sPLA 2

(30) Priority: 01.05.1998 US 83873 P
(43) Date of publication of application: 07.02.2001
(73) Proprietor: ELI LILLY AND COMPANY, Indianapolis, Indiana 46285 (US)
(72) Inventor: DENNEY, Michael, Lyle, Franklin, IN 46131 (US); MORIN, John, Michael, Jr., Brownsburg, IN 46112 (US); SALL, Daniel Jon, Greenwood, IN 46143 (US); SAWYER, Jason, Scott, Indianapolis, IN 46220 (US)
(74) Representative: Kingsbury, Oliver William
(86) International application number: PCT/US1999/008538
(87) International publication number: WO 1999/056752

(56) References cited:
- EP-A- 0 675 110
- WO-A-00/37022
- DATABASE CAPLUS ON STN, Accession No. 1999:297295, No. 130:311696, DENNY et al., "Preparation of Isopropyl Ester Prodrugs of Indole sPLA2 Inhibitors";& WO 9921545 A, 06 May 1999.

## Description

### FIELD OF THE INVENTION

This invention relates to sPLA₂ inhibitor compounds having high bioavailability.

### BACKGROUND OF THE INVENTION

The structure and physical properties of human non-pancreatic secretory phospholipase A₂ (hereinafter called, "sPLA₂") has been thoroughly described in two articles, namely, "Cloning and Recombinant Expression of Phospholipase A₂ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; *The Journal of Biological Chemistry,* Vol. 264, No. 10, Issue of April 5, pp. 5335-5338, 1989; and "Structure and Properties of a Human Non-pancreatic Phospholipase A₂" by Kramer, Ruth M.; Hession, Catherine; Johansen, Berit; Hayes, Gretchen; McGray, Paula; Chow, E. Pingchang; Tizard, Richard; and Pepinsky, R. Blake; *The Journal of Biological Chemistry,* Vol. 264, No. 10, Issue of April 5, pp. 5768-5775, 1989; the disclosures of which are incorporated herein by reference.

It is believed that sPLA₂ is a rate limiting enzyme in the arachidonic acid cascade which hydrolyzes membrane phospholipids. Thus, it is important to develop compounds which inhibit sPLA₂ mediated release of fatty acids (e.g., arachidonic acid) and are highly bioavailable in mammals, especially humans. Such compounds are of value in general treatment of conditions induced and/or maintained by overproduction of sPLA_{2;} such as septic shock, adult respiratory distress syndrome, pancreatitis, trauma, bronchial asthma, allergic rhinitis, rheumatoid arthritis, etc.

Therapeutic agents that may be given orally are, in general, greatly preferred and have enhanced commercial potential because of their inherent ease of use.

U.S. Patent No. 5,654,326 describes certain indole type sPLA₂ inhibitors and related ester prodrugs. In particular, this patent exemplifies the methyl ester of ((3-(2-amino-1,2-dioxoethyl)-2-methyl-1-(phenylmethyl)-1H-indol-4-yl)oxy)acetic acid.

It is desirable to develop more highly available sPLA₂ inhibitors, particularly those suitable for oral administration.

### SUMMARY OF THE INVENTION

This invention is the novel compound,((3-(2-amino-1,2-dioxoethyl)-2-methyl-1-(phenylmethyl)-1H-indol-4-yl)oxy)acetic acid N-morpholino ethyl ester; which is highly bioavailable by oral administration.

This invention is a pharmaceutical formulation comprising ((3-(2-amino-1,2-dioxoethyl)-2-methyl-1-(phenylmethyl)-1H-indol-4-yl)oxy)acetic acid, N-morpholino ethyl ester in combination with a carrier or diluent.

This invention also relates to the compound ((3-(2-amino-1,2-dioxoethyl)-2-ethyl-1-(phenylmethyl) -1H-indol-4-yl)oxy) acetic acid N-morpholino ethyl ester.

### DETAILED DESCRIPTION OF THE INVENTION THE 1H-INDOLE-3-GLYOXYLAMIDE COMPOUND OF THE INVENTION:

The compound of the invention((3-(2-amino-1,2-dioxoethyl)-2-methyl-1-(phenylmethyl)-1H-indol-4-yl)oxy)acetic acid, N-morpholino ethyl ester;
is represented by the structural formula (I);

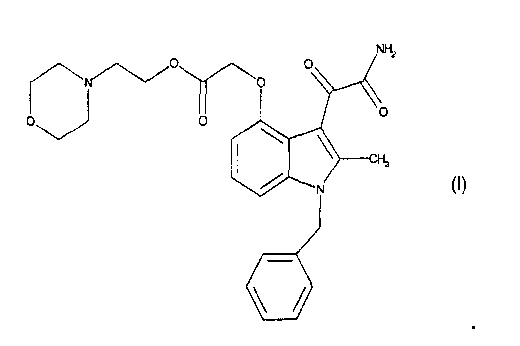

The N-morpholino ethyl ester (I) is an ester form of known sPLA₂ inhibitor ((3-(2-amino-1,2-dioxoethyl)-2-methyl-1-(phenylmethyl)-1H-indol-4-yl)oxy)acetic acid, represented by the structural formula (II), below; 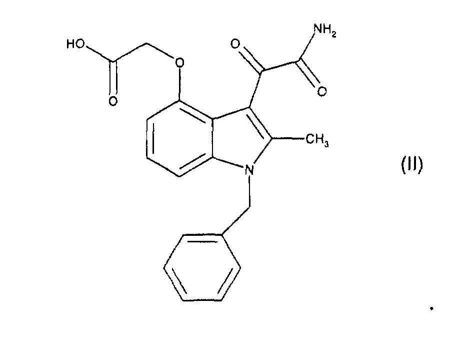 The compound of formula (II) is described in Example 1 of U.S. Patent No. 5,654,326 and European Patent Application No. 95302166.4, Publication No. 0 675 110 (publ., 4 October 1995).

It is a discovery of this invention that the compound of formula (I) is highly bioavailable upon oral administration compared to other sPLA₂ inhibitors.

### SYNTHESIS OF THE COMPOUND OF THE INVENTION:

The synthesis of ((3-(2-amino-1,2-dioxoethyl)-2-methyl-1-(phenylmethyl)-1H-indol-4-yl)oxy)acetic acid, N-morpholino ethyl ester (compound of formula I, supra.) uses as starting material ((3-(2-amino-1,2-dioxoethyl)-2-methyl-1-(phenylmethyl)-1H-indol-4-yl)oxy)acetic acid, or a salt thereof (compound of formula II, supra.). This starting material may be prepared by the reaction schemes or method of Example 1 of U.S. Patent No. 5,654,326 (the disclosure of which is incorporated herein by reference). Similar methods are shown in European Patent Application No. 95302166.4, Publication No. 0 675 110 (publ., 4 October 1995). Other conventional methods may also be used for preparing the starting material. Procedures useful for the synthesis of the compound of this invention are specified in Example 1 set out below:

### Example 1

Preparation of ((3-(2-Amino-1,2-dioxoethyl)-2-methyl-1-(phenylmethyl)-1H-indol-4-yl)oxy)acetic acid, N-morpholino ethyl ester, a compound represented by the formula:

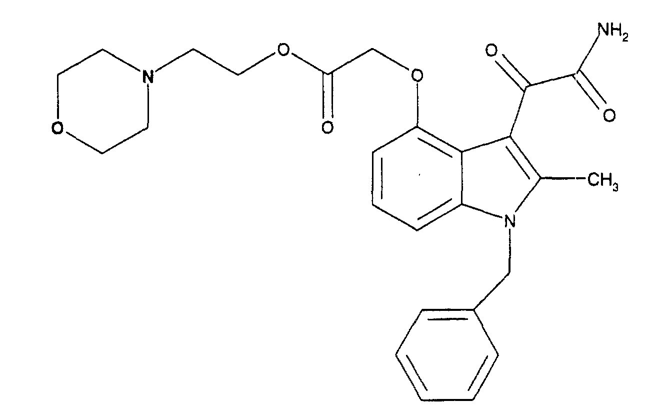

### Part A. Preparation of N-tert-butoxycarbonyl-3-methoxy-2-methylaniline.

A solution of 44.4g (344 mmol) of 3-methoxy-2-methylaniline and 75g (344 mmol)of di-tert-butyl dicarbonate in 400 mL of THF was heated to maintain reflux for 4 hours. After concentrating at reduced pressure, the residue was taken up in ethyl acetate, washed with 1N citric acid, water and dried (MgSO₄). After removing the solvent at reduced pressure, the residue was crystallized from hexane to give 64.5g (84% yield) of N-tert-butoxycarbonyl-3-methoxy-2-methylaniline, mp, 56-57°C.

| Analysis for C₁₃H₁₉NO₃: | | | |
|---|---|---|---|
| Calculated | C, 65.80; | H, 8.07; | N, 5.90 |
| Found | C, 63.32; | H, 7.83; | N, 5.56. |

### Part B. Preparation of 4-Methoxy-2-methyl-1H-indole.

A solution of 280 mL (0.36 mol) of 1.3M sec-butyl lithium in cyclohexane was added slowly to N-tert-butoxycarbonyl-3-methoxy-2-methylaniline (43g, 0.18 mol) in 300 mL of THF keeping the temperature below -40°C with a dry ice-ethanol bath. The bath was removed and the temperature allowed to rise to -20°C and then the bath replaced. After the temperature had cooled to -60°C, 18.5g (0.18 mol) of N-methoxy-N-methylglyoxylamide in an equal volume of THF was added dropwise. The reaction mixture was stirred 1 hour, the cooling bath removed and stirred an additional 1 hour. It was then poured into a mixture of 600 mL of ether and 600 mL of 1N HCl. The organic layer was separated, washed with water, dried over MgSO₄, and concentrated at reduced pressure to give 39.5g of a mixture of 1-(2-(tert-butoxycarbonylamino)-6-methoxyphenyl)-2-propanone and starting anilide. This mixture was dissolved in 100 mL of methylene chloride and 40 mL of trifluoroacetic acid and stirred for a total of 26 hours. The mixture was washed with water, dried(MgSO₄) and concentrated at reduced pressure. The residue was chromatographed on silica gel eluting with 20% EtOAc/hexane to give on crystallization from CH₂Cl₂/hexane 13.9g of 4-methoxy-2-methyl-1H-indole, mp, 80-86°C.

| Analysis for C₁₀H₁₁NO: | | | |
|---|---|---|---|
| Calculated | C, 74.51; | H, 6.88; | N, 8.69 |
| Found | C, 74.41; | H, 7.08; | N, 8.47. |

### Part C. Preparation of 4-Methoxy-2-methyl-1-(phenylmethyl)-1H-indole.

4-Methoxy-2-methyl-1H-indole (1g, 6.2 mmol) was added to 248 mg (6.2 mmol) of 60% sodium hydride/mineral oil (washed with hexane before adding DMF) in 15 mL of DMF and after stirring for 0.5 hour, 0.74 mL (6.2 mmol) of benzyl bromide was added. The mixture was stirred at room temperature for 18 hours, diluted with water and extracted with ethyl acetate. The ethyl acetate solution was washed with brine, dried (MgSO₄) and after concentrating at reduced pressure, the residue was chromatographed on silica gel eluting with 20% EtOAc/hexane to give 1.3g(84% yield) of 4-methoxy-2-methyl-1-(phenylmethyl)-1H-indole, melting at 96-116°C.

| Analyses for C₁₇H₁₇NO: | | | |
|---|---|---|---|
| Calculated | C, 81.24; | H, 6.82; | N, 5.57 |
| Found | C, 81.33; | H, 6.74; | N, 5.29. |

### Part D. Preparation of 4-Hydroxy-2-methyl-1-(phenylmethyl)-1H-indole.

A solution of 1.25g (5 mmol) of 4-methoxy-2-methyl-1-(phenylmethyl)-1H-indole and 20 mL of 1M BBr₃/CH₂Cl₂ in 50 mL of methylene chloride was stirred at room temperature for 5 hours and concentrated at reduced pressure. The residue was dissolved in ethyl acetate, washed with brine and dried (MgSO₄). After concentrating at reduced pressure, the residue was chromatographed on silica gel eluting with 20% EtOAc/hexane to give 577mg (49% yield) of 4-hydroxy-2-methyl-1-(phenylmethyl)-1H-indole, 125-127°C.

| Analyses for C₁₆H₁₅NO: | | | |
|---|---|---|---|
| Calculated | C, 80.98; | H, 6.37; | N, 5.90 |
| Found | C, 80.76; | H, 6.26; | N, 5.80. |

### Part E. Preparation of ((2-Methyl-1-(phenylmethyl)-1H-indol-4-yl)oxy)acetic acid methyl ester.

4-Hydroxy-2-methyl-1-(phenylmethyl)-1H-indole (530mg, 2.2 mmol) was added to 88mg (2.2 mmol) of 60% NaH/mineral oil in 20 mL of DMF and the mixture stirred for 0.67 hours. Then, 0.21 mL (2.2 mmol) of methyl bromoacetate was added and stirring maintained for 17 hours. The mixture was diluted with water and extracted with ethyl acetate. The ethyl acetate solution was washed with brine, dried (MgSO₄), and concentrated at reduced pressure. The residue was chromatographed on silica gel eluting with 20% EtOAc/hexane to give 597mg (88% yield) of ((2-methyl-1-(phenylmethyl)-1H-indol-4-yl)oxy)acetic acid methyl ester, 140-143°C.

| Analyses for C₁₉H₁₉NO₃: | | | |
|---|---|---|---|
| Calculated | C, 73.77; | H, 6.19; | N, 4.53 |
| Found | C, 74.01; | H, 6.23; | N, 4.32. |

### Part F. Preparation of ((3-(2-Amino-1,2-dioxoethyl)-2-methyl-1-(phenylmethyl)-1H-indol-4-yl)oxy)acetic acid methyl ester.

Oxalyl chloride (0.16 mL, 1.9 mmol) was added to 582mg (1.9 mmol) of ((2-methyl-1-(phenylmethyl)-1H-indol-4-yl)oxy)acetic acid methyl ester in 10 mL of methylene chloride and the mixture stirred for 1.5 hours. The mixture was concentrated at reduced pressure and residue taken up in 10 mL of methylene chloride. Anhydrous ammonia was bubbled in for 0.25 hours, the mixture stirred for 1.5 hours and evaporated at reduced pressure. The residue was stirred with 20 mL of ethyl acetate and the mixture filtered. The filtrate was concentrated to give 672mg of a mixture of ((3-(2-amino-1,2-dioxoethyl)-2-methyl-1-(phenylmethyl)-1H-indol-4-yl)oxy)acetic acid, methyl ester and ammonium chloride, mp 202-215°C.

### Part G. Preparation of ((3-(2-Amino-1,2-dioxoethyl)-2-methyl-1-(phenylmethyl)-1H-indol-4-yl)oxy)acetic acid.

A mixture of 660mg (1.7 mmol) of ((3-(2-amino-1,2-dioxoethyl)-2-methyl-1-(phenylmethyl)-1H-indol-4-yl)oxy)acetic acid methyl ester and 10 mL of 1N NaOH in 30 mL of methanol was heated to maintain reflux for 1 hour, cooled to room temperature and stirred for 0.5 hour. The mixture was concentrated at reduced pressure and the residue taken up in EtOAc/water. The aqueous layer was separated, made acidic to pH 2-3 with 1N HCl and extracted with EtOAc. On concentrating the EtOAc solution, 431mg (69% yield) of ((3-(2-amino-1,2-dioxoethyl)-2-methyl-1-(phenylmethyl)-1H-indol-4-yl)oxy)acetic acid crystallized, melting at 218-220°C.

| Analyses for C₂₀H₁₈N₂O₅: | | | |
|---|---|---|---|
| Calculated | C, 65.57; | H, 4.95; | N, 7.65 |
| Found | C, 63.31; | H, 4.79; | N, 6.91. |

### Part H. Preparation of ((3-(2-amino-1,2-dioxoethyl)-2-methyl-1-(phenylmethyl)-1H-indol-4-yl)oxy)acetic acid N-morpholino ethyl ester.

The compound of the present invention may be formed by the reaction of 4-(2-chloroethyl)morpholine hydrochloride (available from Aldrich Chemical Co., Milwaukee, Wisconsin USA, Item No. C4,220-3) and suitable base preferably Cs₂CO₃; and ((3-(2-amino-1,2-dioxoethyl)-2-methyl-1-(phenylmethyl)-1H-indol-4-yl)oxy)acetic acid, sodium salt in a suitable solvent, preferably dimethylformamide. The slurry should be heated to 60°C or other appropriate temperature until a solution is formed. Heating should continued until the reaction is complete. The reaction mixture should be worked up to isolate the product using conventional organic laboratory techniques.

### Assay 1

Cynomolgus monkeys were used in a single dose pharmacokinetic study. The monkeys (3 per treatment) were administered a single oral 10mg/kg dose of one of six indole prodrug compounds including the compound of this invention.

Serial blood samples were obtained up to 24 hours after dose administration. Plasma was analyzed for the corresponding free acid using an LC/MS method. Also area under the curve (auc) values were computed at. 8 and 24 hours.

The purpose of this assay was to evaluate and compare the oral delivery for selected sPLA₂ inhibitors.

### Test Subject:

- Species:: Monkeys
- Strain:: Cynomolgus

### Dose Preparation:

The amount of sPLA₂ inhibitor was corrected for free acid equivalents.

### Vehicle:

Suspension of sPLA₂ inhibitor in 10% Acacia, prepared just prior to dose administration

### Dose Administration:

- Route:: Oral
- Frequency:: Single dose
- Dose:: 10 mg/kg (of the parent acid)
- Dosage Volume:: 5 mL/kg

### Results

### Monkey Pharmacokinetics Study

**Table 1**

| Compound ester type | Cmax (ng/ml) | Tmax hours | AUC (0-8hr) ng*h/ml | AUC (0-24hr) ng*h/ml |
|---|---|---|---|---|
| 1 | 1604 | 2.0 | 5131 | 5425 |
| 2 | 200 | 2.0 | 1356 | 2038 |
| 3 | 213 | 2.0 | 1277 | 1761 |
| 4 | 245 | 2.0 | 1675 | 3404 |
| 5 | 3296 | 2.0 | 11919 | 13161 |
| 6 | 615 | 3.3 | --- | 8730 |
| 1 = ((3-(2-amino-1,2-dioxoethyl)-2-methyl-1-(phenylmethyl)-1H-indol-4-yl)oxy)acetic acid N-morpholino ethyl ester | | | | |
| 2 = ((3-(2-amino-1,2-dioxoethyl)-2-methyl-1-(phenylmethyl)-1H-indol-4-yl)oxy)acetic acid methyl ester | | | | |
| 3 = ((3-(2-Amino-1,2-dioxoethyl)-1-((1,1'-biphenyl)-2-ylmethyl)-2-methyl-1H-indol-4-yl)oxy)acetic acid N,N-diethylglycolamido ester | | | | |
| 4 = ((3-(2-Amino-1,2-dioxoethyl)-2-ethyl-1-(phenylmethyl)-1H-indol-4-yl)oxy)acetic acid ethyl ester | | | | |
| 5 = ((3-(2-Amino-1,2-dioxoethyl)-2-ethyl-1-(phenylmethyl)-1H-indol-4-yl)oxy)acetic acid N-morpholino ethyl ester | | | | |
| 6 = ((3-(2-Amino-1,2-dioxoethyl)-2-ethyl-1-(phenylmethyl)-1H-indol-4-yl)oxy)acetic acid methyl ester | | | | |

### Assay II

The bioavailability of the compound of the invention, ((3-(2-amino-1,2-dioxoethyl)-2-methyl-1-(phenylmethyl)-1H-indol-4-yl)oxy)acetic acid-N-morpholino ethyl ester, was also determined using a Rat Plasma single dose oral Pharmacokinetics Study:
The purpose of this assay was to evaluate and compare the oral delivery for selected sPLA₂ inhibitors.

### Test Subject:

- Species:: Rat
- Strain:: Fischer 344

### Dose Preparation:

The amount of sPLA₂ inhibitor was corrected for free acid equivalents.

### Vehicle:

Suspension of sPLA₂ inhibitor in 10% Acacia, prepared just prior to dose administration

### Dose Administration:

- Route:: Oral
- Frequency:: Single dose
- Dose:: 10 mg/kg (of the parent acid)
- Dosage Volume:: 5 mL/kg
Rats fasted overnight.

### Specimen Collection:

Blood samples (0.8 ml) were obtained at the following times: 0.5, 1, 2, 4, 6 and 8 hours (2 rats/timepoint)

### Data Analysis:

Plasma was assayed by HPLC to measure concentrations of the different sPLA₂ inhibitors (as free acids).

Cmax (maximal plasma concentrations), and AUC values were determined from the mean plasma concentration-time profiles.

**Table 2**

| Compound ester type | Cmax (ng/ml) | AUC (0-8hr) |
|---|---|---|
| 11 | 1094 | 2400 |
| 12 | 79 | 385 |
| 13 | 258 | 1229 |
| 14 | 1199 | 2604 |
| 15 | 612 | 1504 |
| 16 | 259 | 1031 |
| 11 = ((3-(2-amino-1,2-dioxoethyl)-2-methyl-1-(phenylmethyl)-1H-indol-4-yl)oxy)acetic acid N-morpholino ethyl ester (compound of the invention) | | |
| 12 = ((3-(2-Amino-1,2-dioxoethyl)-2-methyl-1-(phenylmethyl)-1H-indol-4-yl)oxy)acetic acid N,N-diethylacetamido ester | | |
| 13 = ((3-(2-Amino-1,2-dioxoethyl)-2-methyl-1-((1-naphthalenyl)methyl)-1H-indol-4-yl)oxy)acetic acid N,N-diethylacetamido ester | | |
| 14 = ((3-(2-Amino-1,2-dioxoethyl)-2-methyl-1-((1-naphthalenyl)methyl)-1H-indol-4-yl)oxy)acetic acid morpholino N-ethyl ester | | |
| 15 = ((3-(2-Amino-1,2-dioxoethyl)-2-ethyl-1-((3-chlorophenyl)methyl)-1H-indol-4-yl)oxy)acetic acid N-morpholino ethyl ester | | |
| 16 = ((3-(2-Amino-1,2-dioxoethyl)-2-ethyl-1-((3-chlorophenyl)methyl)-1H-indol-4-yl)oxy)acetic acid N,N-diethylacetamido ester | | |

## Claims

1. The compound, ((3-(2-amino-2,2-dioxoethyl)-2-methyl-1-(phenylmethyl)-1H-indol-4-yl)oxy)acetic acid N-morpholino ethyl ester.

2. The compound, ((3-(2-amino-1,2-dioxoethyl)-2-ethyl-1-(phenylmethyl)-1H-indol-4-yl)oxy)acetic acid N-morpholino ethyl ester.

3. A pharmaceutical formulation comprising the compound of claim 1 or claim 2 in combination with a carrier or diluent.

4. A compound according to claim 1 or claim 2 for use in therapy.

5. Use of a compound as claimed in claim 1 or claim 2 for the manufacture of a medicament for the treatment of septic shock, adult respiratory distress syndrome, pancreatitis, trauma, bronchial asthma, allergic rhinitis, or rheumatoid arthritis.

## Patentansprüche

1. Verbindung ((3-(2-Amino-1,2-dioxoethyl)-2-methyl-1-(phenylmethyl)-1H-indol-4-yl)oxy)essigsäure-N-morpholinoethylester.

2. Verbindung ((3-(2-Amino-1,2-dioxoethyl)-2-ethyl-1-(phenylmethyl)-1H-indol-4-yl)oxy)essigsäure-N-morpholinoethylester.

3. Pharmazeutische Formulierung, enthaltend die Verbindung nach Anspruch 1 oder Anspruch 2 in Kombination mit einem Träger oder Verdünnungsmittel.

4. Verbindung nach Anspruch 1 oder Anspruch 2 zur Verwendung in der Therapie.

5. Verwendung einer Verbindung nach Anspruch 1 oder Anspruch 2 zur Herstellung eines Arzneimittels für die Behandlung von septischem Schock, Atemnotsyndrom beim Erwachsenen, Pankreatitis, Trauma, Bronchialasthma, allergischer Rhinitis oder rheumatoider Arthritis.

## Revendications

1. Le composé, ester N-morpholino éthylique de l'acide ((3-(2-amino-1,2-dioxoéthyl)-2-méthyl-1-(phénylméthyl)-1H-indol-4-yl)oxy)acétique.

2. Le composé, ester N-morpholino éthylique de l'acide ((3-(2-amino-1,2-dioxoéthyl)-2-éthyl-1-(phénylméthyl)-1H-indol-4-yl)oxy)acétique.

3. Formulation pharmaceutique comprenant le composé selon la revendication 1 ou la revendication 2 en association avec un support ou un diluant.

4. Composé selon la revendication 1 ou la revendication 2 destiné à une utilisation thérapeutique.

5. Utilisation d'un composé tel que revendiqué dans la revendication 1 ou la revendication 2 pour la fabrication d'un médicament destiné au traitement du choc septique, du syndrome de détresse respiratoire de l'adulte, de la pancréatite, d'un traumatisme, de l'asthme bronchique, de la rhinite allergique ou de la polyarthrite rhumatoïde.
